# EUROPEAN PATENT SPECIFICATION

(11) **EP 3 406 245 B1**
(45) Date of publication and mention of the grant of the patent: **09.11.2022**
(21) Application number: 16885486.7
(22) Date of filing: 29.12.2016
(51) Int. Cl.: A61K 31/40, A61K 31/366, A61K 31/505, A61K 31/573, A61P 25/00, A61P 9/10

(54) **JOINT APPLICATION OF STATIN AND GLUCOCORTICOID FOR TREATING CHRONIC SUBDURAL HEMATOMA**
GEMEINSAME ANWENDUNG VON STATIN UND GLUCOCORTICOID ZUR BEHANDLUNG CHRONISCHER SUBDURALER BLUTERGÜSSE
APPLICATION CONJOINTE DE STATINE ET DE GLUCOCORTICOÏDE POUR TRAITER UN HÉMATOME SOUS-DURAL CHRONIQUE

(30) Priority: 20.01.2016 CN 201610035891
(43) Date of publication of application: 28.11.2018
(73) Proprietor: Zhang, Jianning, Tianjin 300052 (CN); Jiang, Rongcai, Tianjin 300052 (CN); Wang, Dong, Tianjin 300052 (CN)
(72) Inventor: ZHANG, Jianning, Tianjin 300052 (CN); JIANG, Rongcai, Tianjin 300052 (CN); WANG, Dong, Tianjin 300052 (CN); QUAN, Wei, Tianjin 300052 (CN)
(74) Representative: Geling, Andrea
(86) International application number: PCT/CN2016/000720
(87) International publication number: WO 2017/124213

(56) References cited:
- WO-A1-2006/123125
- CN-A- 103 203 020
- EL-BARBARY AMAL M ET AL: "Effect of atorvastatin on inflammation and modification of vascular risk factors in rheumatoid arthritis", JOURNAL OF RHEUMATO, TORONTO, ONT : JOURNAL OF RHEUMATOLOGY, vol. 38, no. 2, 1 January 2011 (2011-01-01), pages 229-235, XP009192998, ISSN: 0315-162X, DOI: 10.3899/JRHEUM.100582
- JINHAO HUANG ET AL: "Treatment of Relapsed Chronic Subdural Hematoma in Four Young Children with Atorvastatin and Low-dose Dexamethasone", PHARMACOTHERAPY : THE JOURNAL OF HUMAN PHARMACOLOGY AND DRUG THERAPY, vol. 39, no. 7, 13 July 2019 (2019-07-13), pages 783-789, XP055609933, US ISSN: 0277-0008, DOI: 10.1002/phar.2276
- JAMEEL, A. et al.: "Statin Modulation of Human T- Cell Proliferation, IL -1beta and IL -17 Production, and IFN- y T Cell Expression: Synergy with Conventional Immunosuppressive Agents", INTERNATIONAL JOURNAL OF INFLAMMATION, vol. 2013, 1 January 2013 (2013-01-01), pages 1-11, XP055558756, ISSN: 2090-8040, DOI: 10.1155/2013/434586
- JAMEEL, A. et al.: "Statin Modulation of Human T- Cell Proliferation, IL -1beta and IL -17 Production, and IFN- y T Cell Expression: Synergy with Conventional Immunosuppressive Agents", INTERNATIONAL JOURNAL OF INFLAMMATION, vol. 2013, 1 January 2013 (2013-01-01), pages 1-12, XP055558756, ISSN: 2090-8040, DOI: 10.1155/2013/434586
- SHEN, BIN et al.: "Prescription Analysis of Drug Combination of Three Statins", Yiyao Daobao HERALD OF MEDICINE, vol. 29, no. 11, 30 November 2010 (2010-11-30), pages 1505-1507, XP009512078, ISSN: 1004-0781
- HE, YUNWEN et al.: "Observation on Clinical Curative Effect of Dexamethasone in Treatment of Chronic Subdural Hematoma", JOURNAL OF NORTH PHARMACY, vol. 12, 1 August 2015 (2015-08-01), pages 42-43, XP9512475, ISSN: 1672-8351
- YANG WUYANG ET AL: "Chronic Subdural Hematoma: Epidemiology and Natural History.", NEUROSURGERY CLINICS OF NORTH AMERICA APR 2017, vol. 28, no. 2, April 2017 (2017-04), pages 205-210, ISSN: 1558-1349
- KOLIAS ANGELOS G ET AL: "Chronic subdural haematoma: modern management and emerging therapies.", NATURE REVIEWS. NEUROLOGY OCT 2014, vol. 10, no. 10, October 2014 (2014-10), pages 570-578, ISSN: 1759-4766

## Description

### FIELD OF THE INVENTION

The present invention pertains to the technical field of pharmaceutical supplies containing effective ingredients, and in particular, relates to combination use of statins and dexamethasone or structural analogues thereof for the treatment of chronic subdural hematoma.

### BACKGROUND OF THE INVENTION

People have been exploring and studying the chronic subdural hematoma (CSDH) for more than a century, and the earliest study may be dated back to more than 150 years ago. It is documented that Virchow firstly studied the formation mechanism of the chronic subdural hematoma, and Virchow firstly depicted this disease and proposed the hypothesis that the inner dura mater inflammatory hemorrhage is the cause to the CSDH, that is, Pachymeningitis Theory. Afterwards, through a series of studies of pathological examinations and electron microscopy observation and so on, it has been found that the chronic subdural hematoma capsule contains a large number of abnormal sinusoid lacunar vessels. Since then, "newborn capsules' repeated bleeding theory" for the subdural hematoma is gradually concentrated by the academic attention.

In recent years, fundamental studies on the chronic subdural hematoma have identified that the disease is an inflammatory angiogenic disease, and thus this view point has been increasingly accepted by the public. The levels of inflammatory cytokines IL-6, IL-8 and IL-10 in the hematoma fluid are significantly increased relative to those in the peripheral blood, the levels of IL-6 and VEGF in the hematoma fluid of the recurrence patients are significantly increased relative to those in the non-recurrence patients, and the VEGF expression in the adventitia of the hematoma cavity is also significantly increased. The levels of these cytokines are closely related to the recurrence of the hematoma. At the same time, the researchers have also found that angiogenesis-related factors PIGF, VEGF, bFGF, MMP-2 and MMP-9 in the hematoma fluid are significantly increased, and Ang-1 and Ang-2 mRNA promoting angiogenesis in the adventitia of the hematoma are increased. A decrease of the proportion thereof indicates an increase of the generation of new vessels. The combination of the inflammatory factors and the angiogenic factors may be a key factor in the formation of CSDH.

It is well recognized that surgical treatment of the chronic subdural hematoma is currently the most effective treatment, especially in the treatment of the patients with large amounts of hematoma, obvious clinical symptoms and a midline shift greater than 5 mm. Through the surgical operation, intracranial hypertension and brain hernia may be quickly relieved. In addition to the surgical treatment, other conservative treatments of the chronic subdural hematoma mainly include: simply lying still and guarding, reducing intracranial pressure by mannitol dehydration, anti-epileptic treatment, glucocorticoid therapy, angiotensin-converting enzyme inhibitor, and even radiation therapy and the like.

In recent years, a large number of reports reveal studies on the treatment of chronic subdural hematoma using hormones on a larger scale. For example, in the year of 2005, Sun reported that, after being treated with dexamethasone for 21 days (4 mg/6h), 23 of the 26 elderly patients, who were intolerant to the surgical operation and had complications, have a better prognosis (84%), and the recurrences are significantly reduced for the patients after they were continuously administered with dexamethasone (DXM) for 2 weeks following the surgical operation. Likewise, individual retrospective studies performed by Dran and Berghause have demonstrated that the death risk of the group postoperatively administered with dexamethasone is reduced by 3 folds as compared with the non-administered group, and perioperative administration of dexamethasone may reduce the postoperative recurrence. A perspective study using dexamethasone by Delgado-López *et al.* has also proved that it is effective to treat the chronic subdural hematoma with hormone. In the year of 2014, a randomized double-blind controlled China-Austria multi-center clinical trial (DRESH trial) planned to enroll 820 patients, and the final result of the study would further clarify whether perioperative administration of dexamethasone can reduce postoperative CSDH recurrence. A randomized double-blind placebo-controlled trial with an experimental group and a control group each involving 10 patients (registration number: NCT02362321) was completed in Canada, and as a result, 1 patient in the group of dexamethasone and 3 patients in the group of placebo are transferred to the surgical treatment. However, in the above trials, a large amount of hormones is used, and a large dose of dexamethasone may easily cause obesity, gastrointestinal damage and other steroid-related complications. Therefore, the hormone therapy has not been promoted, and most applications thereof are limited to auxiliary medication after surgery. Chinese Patent No. 201210014181.4, entitled with "USE OF STATINS IN PREPARING A DRUG FOR THE TREATMENT OF CHRONIC SUBDURAL HEMATOMA", has disclosed that the statins may obviously promote the absorption of chronic subdural hematoma, such that some patients can achieve the purpose of hematoma absorption through conservative treatment with drugs, thereby avoiding surgical treatment. However, this therapy works slowly, and it takes up to six months, or at least one month to absorb the hematoma after the patients receive the treatment, causing confusion to some patients.

The document CN103203020 describes application of a statin for treating chronic subdural hematoma. The document by He Yunwen et al (2015), Journal of North Pharmacy, vol.12, pp.42-43 describes application of dexamethasone for treating chronic subdural hematoma.

### SUMMARY OF THE INVENTION

The invention is as defined in the claims.

The present invention intends to solve the problems that the period for currently treating chronic subdural hematoma by using statin in clinical is long, and the use of a large amount of hormones causes a lot of complications to patients, and intended to address the technical prejudice hold by a person skilled in the art against the dosages of hormones.

The present invention provides a combination of statin and glucocorticoid for use in a method of treating chronic subdural hematoma.

Described herein, but not encompassed by the present invention, is the use of statins and adrenocortical hormones for preparing a pharmaceutical product for the treatment of chronic subdural hematoma.

Preferably, the statin is one or a combination of atorvastatin, simvastatin and rosuvastatin.

Preferably, the glucocorticoid is one or a combination of dexamethasone, prednisone, methylprednisolone and hydrocortisone.

More preferably, the glucocorticoid is dexamethasone or prednisone.

Preferably, a mass ratio of the statin to the glucocorticoid in an equivalent dose is 40:3. More preferably, the statin is atorvastatin, and the glucocorticoid is dexamethasone, wherein a mass ratio of atorvastatin to dexamethasone is 40:3.

Administration doses of the statin and the glucocorticoid are respectively as follows:
30 mg/day, 2.25 mg/day; or 20 mg/day, 1.5 mg/day; or 10 mg/day, 0.75 mg/day; wherein the doses can be administered as one administration per day or two to three administrations per day.

Preferably, the administration doses of the statin and the glucocorticoid are respectively as follows:
administration in the first week: 30 mg/day, 2.25 mg/day;
administration in the second to the third week: 20 mg/day, 1.5 mg/day;
administration in the fourth week: 10 mg/day, 0.75 mg/day.

Described herein, but not encompassed by the present invention, is the provision of a pharmaceutical composition, comprising: a first active ingredient in a therapeutically effective amount, the first active ingredient being statins; and a second active ingredient in a therapeutically effective amount, the second active ingredient being adrenocortical hormones, and preferably, dexamethasone and a structural analogue thereof. Preferably, the statins are one or a combination of atorvastatin, simvastatin and rosuvastatin.

Preferably, the structural analogue of dexamethasone is dexamethasone or prednisone.

Preferably, a mass ratio of the first active ingredient to the second active ingredient of the pharmaceutical composition in an equivalent dose is 40:3.

The equivalent dose as described herein refers to a relative drug concentration or dose which causes an equivalent effect, and reflects a relationship between the drug effect and the drug dose. The mass ratio of the first active ingredient to the second active ingredient in the equivalent dose being 40:3 is determined based on the administration doses of atorvastatin and dexamethasone. That is, the mass ratio of atorvastatin to dexamethasone is 40:3. Other statins all need to be used in a converted dose equivalent to atorvastatin, and other structural analogues of dexamethasone also need to be used in a converted dose equivalent to dexamethasone.

In clinical use, a conversion ratio of the equivalent dose of the statins is rosuvastatin: atorvastatin: simvastatin= 1:3:4 (a maximum dose of the simvastatin is 40 mg/day).

A conversion ratio of the equivalent dose of the structural analogues of dexamethasone is dexamethasone: prednisone=1:5.

Therefore, in the pharmaceutical composition, a mass ratio of rosuvastatin to dexamethasone is 40:9.

In the pharmaceutical composition, a mass ratio of rosuvastatin to prednisone is 8:9.

In the pharmaceutical composition, a mass ratio of atorvastatin to prednisone is 8:3.

In the pharmaceutical composition, a mass ratio of simvastatin to dexamethasone is 160:9.

In the pharmaceutical composition, a mass ratio of simvastatin to prednisone is 32:9.

In the pharmaceutical composition, administration doses of the statins and the adrenocortical hormones are respectively as follows:
30 mg/day, 2.25 mg/day; or 20 mg/day, 1.5 mg/day; or 10 mg/day, 0.75 mg/day; the doses can be administered as one administration per day or two to three administrations per day.

Preferably, oral doses of atorvastatin and dexamethasone in the pharmaceutical composition are respectively as follows:
administration in the first week: 30 mg/day, 2.25 mg/day;
administration in the second to the third week: 20 mg/day, 1.5 mg/day; or
administration in the fourth week: 10 mg/day, 0.75 mg/day.

Described herein, but not encompassed by the present invention, is the use of the pharmaceutical composition for preparing a pharmaceutical product for the treatment of chronic subdural hematoma.

The present invention achieves the following beneficial effects:
(1) Compared with the use of the statin alone, the combination of the drugs according to the present invention can significantly accelerate the absorption of hematoma, an average time of the hematoma being significantly reduced is about 2 weeks, and an average time of the hematoma disappearing is about 1.5 months, which greatly improves the therapeutic effect achieved by oral administration of the statins alone. Even when the statins treatment is not effective, adding hormones could also achieve satisfactory therapeutic effects without any other adverse effects.
(2) The dosage of the hormones used in the present invention is 1/10 of that in the treatment with a large dose of hormones (16 to 24 mg/d), which greatly reduces the incidence of related complications (gastrointestinal bleeding, ulcer and infection) caused by hormone therapy. In addition, a person skilled in the art would habitually think that the greater the dose of hormones is, the more obvious the therapeutic effect will be. However, the present invention combines the statin with a small dose of dexamethasone, overcomes the technical prejudice, and also achieves unexpected therapeutic effects: the treatment cycle is significantly shortened, and the pains caused by the surgery to the patients are avoided.
(3) The pharmaceutical composition described herein has a good therapeutic effect, broad application prospects and clinical significances.

### BRIEF DESCRIPTION OF THE DRAWINGS

FIG. 1 is a diagram showing the influence of different doses of atorvastatin on the expression amount of VEGF factor in the hematoma capsule of a rat according to the present invention;
FIG. 2 is a diagram showing the influence of different doses of atorvastatin on the expression amount of TGF-β factor in the hematoma capsule of a rat according to the present invention;
FIG. 3 is a diagram showing the influence of different doses of atorvastatin on the expression amount of MMP-9 factor in the hematoma capsule of a rat according to the present invention;
FIG. 4 is a diagram showing the influence of different doses of atorvastatin on the expression amount of Ang-1 factor in the hematoma capsule of a rat according to the present invention;
FIG. 5 is a diagram showing the influence of different doses of atorvastatin on the expression amount of Ang-2 factor in the hematoma capsule of a rat according to the present invention;
FIG. 6 is a diagram showing the influence of different doses of atorvastatin on the ratios of Ang-1/Ang-2 in the hematoma capsule of a rat according to the present invention;
FIG. 7 is a diagram showing the influence of different doses of atorvastatin on the expression amount of VEGF factor in the hematoma cavity of a rat according to the present invention;
FIG. 8 is a diagram showing the influence of different doses of atorvastatin on the expression amount of TGF-β factor in the hematoma cavity of a rat according to the present invention;
FIG. 9 is a diagram showing the influence of different doses of atorvastatin on the expression amount of MMP-9 factor in the hematoma cavity of a rat according to the present invention;
FIG. 10 is an diagram showing the influence of different treatment groups of the present invention on the subcutaneous hematoma volume of a rat;
FIG. 11 is a head CT diagram of a patient before treatment;
FIG. 12 is a head CT diagram of a patient after receiving treatment with a pharmaceutical composition of the present invention for 2 weeks;
FIG. 13 is a head CT diagram of a patient after receiving treatment with a pharmaceutical composition of the present invention for four weeks;
FIG. 14 is a head CT diagram of a patient who is administered with atorvastatin for three months after stopping the pharmaceutical composition according to the present invention previously given for four weeks; and
FIG. 15 is a comparison of head CT diagrams of a patient before and after treatment.

### DETAILED DESCRIPTION OF THE INVENTION

The invention is as defined in the claims.

For further interpreting the technical solutions of the present invention and the technical effects to be achieved thereby, hereinafter the present invention is further described with reference to the accompanying drawings and the exemplary embodiments.

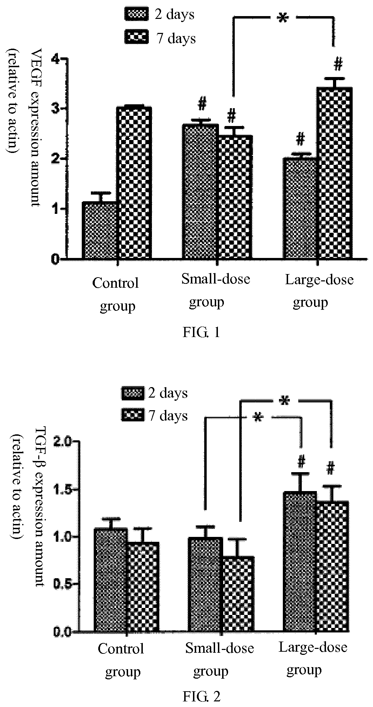

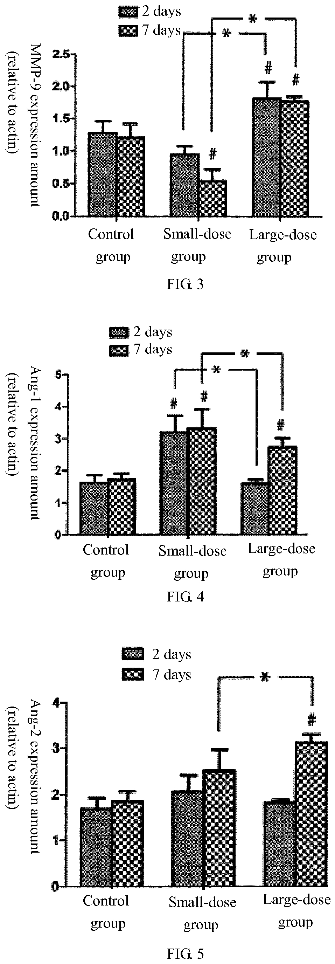

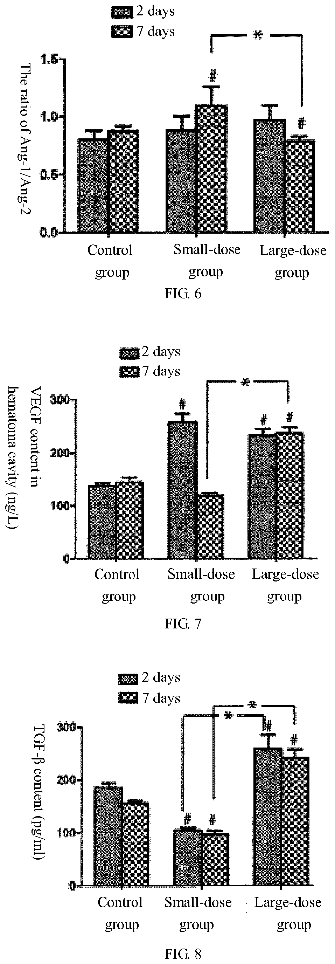

1. A small dose of atorvastatin promotes the absorption of subdural hematoma. Experimental animals were divided into the following three groups by improving subdural modeling of rats and on the basis of varied oral doses of atorvastatin: a control group, a small-dose group (3 mg/kg/day, equivalent to 10-20 mg/day for adults), and a large-dose group (8 mg/kg/day, equivalent to 80-100 mg/day for adults). Dynamic changes of the hematoma volume in the rats were observed by using MRI. HE staining, transmission electron microscopy, immunohistochemistry, flow cytometry, real-time quantitative PCR and ELISA techniques were respectively used to measure physiological structural features of neogenetic hematoma capsular diseases, numbers of endothelial progenitor cells in peripheral blood, neovascularization and densities of smooth muscle-like cells and neogenetic blood vessels, expression changes of the angiogenesis-related factors VEGF, TGF-β, MMP-9 and Ang-1/2 in the peripheral blood and hematoma capsules at different observation points after modeling. The experiment results showed that on day 2, the mRNAs expressions of hematoma capsule VEGF (FIG. 1 and FIG. 7) and Ang1 (FIG. 4) were significantly higher in the small-dose group, whereas the large-dose group showed significantly higher expressions of VEGF, TGF-β and MMP-9 (FIG. 1 to FIG. 3, and FIG. 7 to FIG. 9). At the time of 7 days after the surgery, the capsular vessel-related factors of VEFG and MMP-9 in the small-dose group showed significantly lower expression, and Ang1 and the ratio of Ang-1/Ang-2 (FIG. 4 to FIG. 6) were significantly higher than those of the control group and the large-dose group. However, the large-dose group showed significantly higher expressions of VEGF, TGF-β, MMP-9 and Ang2.

In FIG. 1 to FIG. 9, "#" denotes a statistically significant difference in the small-dose group and the large-dose group as compared with the control group; and "*" denotes a statistically significant difference between the small-dose group and the large-dose group.

Through the experiments, we concluded that the absorption speed of the subdural hematoma was not only related to the number of neogenetic vessels of the hematoma capsule, but also closely related to the mature and stable neovascularization in the capsule; the small-dose atorvastatin accelerated the absorption of the hematoma by inhibiting the local inflammatory reaction in the hematoma cavity, and promoting the formation of mature and stable neovascularization.

2. Atorvastatin in combination with a small dose of dexamethasone promoted the absorption of the subcutaneous hematoma of a rat.

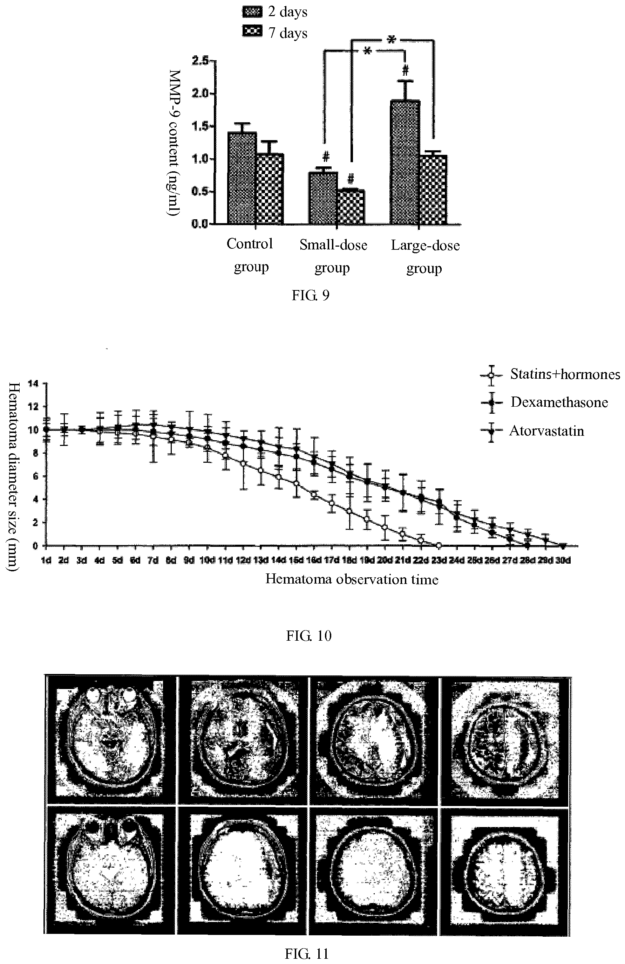

Through the mouse subcutaneous injection model, by intragastrically administering a small dose of atorvastatin, a large dose of dexamethasone, and a small dose of atorvastatin in combination with a small dose of dexamethasone, hematoma changes were dynamically observed. The experiment results showed (see Table 1) that use of the small dose of atorvastatin in combination with the small dose of dexamethasone significantly accelerated the absorption speed of hematoma (FIG. 10); as compared with the group of the small-dose of atorvastatin, the absorption speed of the hematoma in the group of the large-dose of dexamethasone was not significantly improved, whereas the hormone-related complications were significantly increased and the death rate was significantly raised. The small dose of atorvastatin in combination with the small dose of dexamethasone exerted a significant synergistic effect, and further researches on its specific molecular biological level were still needed.

**Table 1 Dynamic changes of hematoma of rats in different treatment groups**

| Item/Group | Animal numbers per group | Death number during treatment | Wound infection | Poor state |
|---|---|---|---|---|
| Small-dose atorvastatin group 3 mg/kg/day | 30 | 2 | 1 | 3 |
| Large-dose dexamethasone group 5 mg/kg/day | 28 | 7 | 4 | 6 |
| Atorvastatin (3 mg/kg/day)+dexamethasone (0.5 mg/kg/day) | 30 | 1 | 1 | 2 |
| Total number | 88 | 10 | 6 | 11 |

3. Pre-clinical experiments showed that use of atorvastatin alone promoted the absorption of hematoma, but the absorption speed of the hematoma was low and some patients were not sensitive to the drug; and use of a small dose of dexamethasone alone exerted a poor hematoma absorption effect.

52 patients were orally administered with atorvastatin alone (in the first week, atorvastatin 10 mg, 3 times per day; in the second to the third week, atorvastatin 10 mg, 2 times per day; in the fourth week, atorvastatin 10 mg, 1 time per day; and after four weeks, 10 mg per day until the hematoma was absorbed or the hematoma was stable without any changes). Before treatment, the patients had a hematoma amount of 55.94±30.58 ml, and after the treatment for one month, patients with a successful conservative treatment had a hematoma amount of 28.29±29.47 ml, an average hematoma absorption rate of 49.43% after one month of administration, and an average administration period of about 3.23 months. 7 patients therein had a poor hematoma absorption effect after being administered with atorvastatin: 4 patients were directly transferred to the surgical treatment to remove hematoma; 3 patients were transferred to the treatment with the group of atorvastatin + a small dose of dexamethasone, and the hematoma thereof was stable and gradually absorbed (see Table 2 for details).

**Table 2 Conditions of 52 patients after being treated with atorvastatin alone via oral administration**

| Serial number | Age | Hematoma amount before entering the group (ml) | Hematoma amount in the fourth week (ml) | Reduced hematoma amount in the fourth week (ml) |
|---|---|---|---|---|
| 1 | 79 | 80 | 40 | 40 |
| 2 | 60 | 27 | 6 | 21 |
| 3 | 75 | 60 | 15 | 45 |
| 4 | 83 | 67.5 | 30 | 37.5 |
| 5 | 75 | 50 | 20 | 30 |
| 6 | 67 | 40 | 0 | 40 |
| 7 | 70 | 30 | 20 | 10 |
| 8 | 25 | 15 | 0 | 15 |
| 9 | 81 | 70.4 | 30 | 40.4 |
| 10 | 46 | 57.5 | 0 | 57.5 |
| 11 | 68 | 60 | 30 | 30 |
| 12 | 79 | 45 | 0 | 45 |
| 13 | 81 | 49.5 | 27 | 22.5 |
| 14 | 66 | 23.75 | 17.5 | 6.25 |
| 15 | 69 | 59.85 | 20.5 | 39.35 |
| 16 | 63 | 35 | 0 | 35 |
| 17 | 68 | 48 | 0 | 48 |
| 18 | 74 | 16 | 4 | 12 |
| 19 | 59 | 72 | 48 | 24 |
| 20 | 37 | 29.7 | 10.6 | 19.1 |
| 21 | 80 | 46.2 | 23.4 | 22.8 |
| 22 | 79 | 89 | 24 | 65 |
| 23 | 79 | 89 | Surgical treatment | Surgical treatment |
| 24 | 70 | 107.90 | 146.85 | Surgical treatment |
| 25 | 68 | 177.45 | 138.60 | 38.85 |
| 26 | 58 | 52.5. | 15.00 | 37.5 |
| 27 | 74 | 66.0 | 29.25 | 36.75 |
| 28 | 66 | 73.13 | 81.90 | -8.775 |
| 29 | 68 | 47.25 | 38.00 | 9.25 |
| 30 | 83 | 123.75 | 30.00 | 93.75 |
| 31 | 70 | 83.16 | 36.00 | 47.16 |
| 32 | 79 | 131.04 | The effect was poor and hormonal treatment was used | The effect was poor and hormonal treatment was used |
| 33 | 66 | 73.13 | 35.00 | 38.125 |
| 34 | 58 | 55.00 | 40.00 | 15 |
| 35 | 56 | 45.00 | 30.00 | 15 |
| 36 | 50 | 33.00 | 25.00 | 8 |
| 37 | 59 | 42.35 | 26.00 | 16.35 |
| 38 | 66 | 55.00 | 40.00 | 15 |
| 39 | 62 | 40.00 | 38.00 | 2 |
| 40 | 64 | 65.00 | 45.00 | 20 |
| 41 | 68 | 35.00 | 30.00 | The effect was poor and hormonal treatment was used |
| 42 | 56 | 30.00 | 5.00 | 25 |
| 43 | 58 | 40.00 | 18.00 | 22 |
| 44 | 75 | 42.00 | 25.00 | 17 |
| 45 | 72 | 35.00 | 10.00 | 25 |
| 46 | 68 | 35.00 | 5.00 | 30 |
| 47 | 78 | 68.00 | Surgical treatment | Surgical treatment |
| 48 | 75 | 78.00 | Surgical treatment | Surgical treatment |
| 49 | 69 | 42.00 | 35.00 | 7 |
| 50 | 50 | 26.00 | 0.00 | 26 |
| 51 | 45 | 29.00 | 41.00 | -12 |
| 52 | 32 | 18.00 | The effect was poor and hormonal treatment was used | The effect was poor and hormonal treatment was used |
| Average value | 65.9 | 55.94 ml | 28.29 ml | 49% (hematoma absorption rate) |

15 patients were orally administered with a small dose of dexamethasone tablets alone (in the first week, dexamethasone 0.75 mg, 3 times per day; in the second to the third week, dexamethasone 0.75 mg, 2 times per day; in the fourth week, dexamethasone 0.75 mg, 1 time per day; after four weeks, 0.75 mg of dexamethasone per day until the hematoma was absorbed or the hematoma was stable without any changes). Before treatment, the patients had a hematoma amount of 79.97±21.00 ml, and the conservative and successful patients had an amount of hematoma and after the treatment for one month, patients with a successful conservative treatment had a hematoma amount of 50.86±24.52 ml, an average hematoma absorption rate of 24.71% after one month of administration. No therapeutic effect was exerted for 5 patients therein after being administered with a small dose of dexamethasone alone, and these 5 patients were transferred to surgical treatment for removing the hematoma (see Table 3).

**Table 3 Conditions of 15 patients after being treated with a small dose of dexamethasone**

| Serial number | Age | Hematoma amount before entering the group (ml) | Hematoma amount in the fourth week (ml) | Reduced hematoma amount in the fourth week (ml) | Hematoma absorption rate in the fourth week (%) |
|---|---|---|---|---|---|
| 1 | 66 | 75.04 | Surgical treatment | Surgical treatment | Surgical treatment |
| 2 | 58 | 61.88 | 40.95 | 20.93 | 0.34 |
| 3 | 56 | 105.00 | 80.00 | 25.00 | 0.24 |
| 4 | 50 | 82.23 | Surgical treatment | Surgical treatment | Surgical treatment |
| 5 | 59 | 75.94 | 36.30 | 39.64 | 0.52 |
| 6 | 66 | 56.62 | 32.73 | 23.90 | 0.42 |
| 7 | 62 | 62.40 | Surgical treatment | Surgical treatment | Surgical treatment |
| 8 | 64 | 104.65 | 96.53 | 8.13 | 0.08 |
| 9 | 68 | 78.30 | 39.88 | 38.43 | 0.49 |
| 10 | 56 | 54.33 | 30.25 | 24.08 | 0.44 |
| 11 | 58 | 117.00 | Surgical treatment | Surgical treatment | Surgical treatment |
| 12 | 75 | 71.40 | 35.00 | 36.40 | 0.51 |
| 13 | 72 | 107.25 | Surgical treatment | Surgical treatment | Surgical treatment |
| 14 | 68 | 93.60 | 80.00 | 13.60 | 0.15 |
| 15 | 78 | 54.00 | 37.00 | 17.00 | 0.31 |
| Average value | | 79.97 ml | 50.86 ml | 24.71 ml | 35% |

The experiment results showed that use of statins alone achieved the effects of regulating the local capsular angiogenesis of hematoma, promoting the maturation of neogenetic capsular vessels, stabilizing vascular intima, inhibiting local abnormal inflammatory reactions, and reducing vascular exudation and the like. However, the effect of inhibiting inflammation was weak, and use of glucocorticoids alone still failed to achieve the effects of vascularization and intima stabilization. The combination of the two drugs could achieve a synergistic effect, remarkably shortened the onset time of the drugs, accelerated the absorption of hematoma, and reduced the adverse complications caused by the hormone.

4. Specific schemes of the treatment for chronic subdural hematoma by using the statins in combination with a small dose of dexamethasone

In addition to receiving the conventional treatment, the patients took the pharmaceutical composition according to the following specific method (the clinical test results were listed in Table 4):
in the first week: atorvastatin 10 mg + dexamethasone 0.75 mg, 3 times per day;
in the second to the third week: atorvastatin 10 mg + dexamethasone 0.75 mg, 2 times per day; and
in the fourth week: atorvastatin 10 mg + dexamethasone 0.75 mg, 1 time per day.

**Table 4 Conditions of 11 patients after the treatment with a combination of the statins and a small dose of dexamethasone**

| Serial number | Age | Hematoma amount before entering the group (ml) | Hematoma amount in the second week (ml) | Hematoma absorption rate in the second week (%) | Hematoma amount in the fourth week (ml) | Hematoma absorption rate in the fourth week (%) | Hematoma amount in the third month (ml) | Hematoma absorption rate in the third month (%) |
|---|---|---|---|---|---|---|---|---|
| 1 | 20 | 61.88 | 40.95 | 0.34 | 34.65 | 0.44 | 12.00 | 0.81 |
| 2 | 90 | 105.00 | 40.00 | 0.62 | 0 | 1.00 | 0 | 1.00 |
| 3 | 72 | 83.23 | 20.00 | 0.76 | 0 | 1.00 | 0 | 1.00 |
| 4 | 77 | 75.94 | 36.30 | 0.52 | 16.20 | 0.79 | 0 | 1.00 |
| 5 | 75 | 56.62 | 32.73 | 0.42 | 16.80 | 0.70 | 0 | 1.00 |
| 6 | 56 | 104.65 | 96.53 | 0.08 | 77.19 | 0.26 | 20.00 | 0.81 |
| 7 | 82 | 78.30 | 39.88 | 0.49 | 0 | 1.00 | 0 | 1.00 |
| 8 | 61 | 54.33 | 30.25 | 0.44 | 28.79 | 0.47 | 18.65 | 0.66 |
| 9 | 66 | 117.00 | 80.00 | 0.32 | 18.00 | 0.85 | 0 | 1.00 |
| 10 | 82 | 71.40 | 35.00 | 0.51 | 10.00 | 0.86 | 0 | 1.00 |
| 11 | 78 | 54.00 | 37.00 | 0.31 | 25.00 | 0.54 | 8.00 | 0.85 |
| Average value | 69 | 78.30 | 44.42 | 44% | 20.60 | 72% | 5.33 | 95% |

The clinical experiments showed that after the 11 patients were subjected to the treatment with the combination of statins and a small dose of dexamethasone, the hematoma was quickly absorbed, and no obvious complications and drug adverse reactions occurred.

### 5. Typical case exhibition

The patient with serial number 9 in Table 4 had a head trauma for more than 1 month. After 2 days of limited physical activity, a subdural hematoma was found via an examination, the hematoma amount thereof was about 117 ml, and the midline shifted towards the right significantly (see FIG. 11). After the treatment with the combination of atorvastatin and a small dose of dexamethasone, the hematoma was gradually absorbed, and the surgical treatment was avoided.

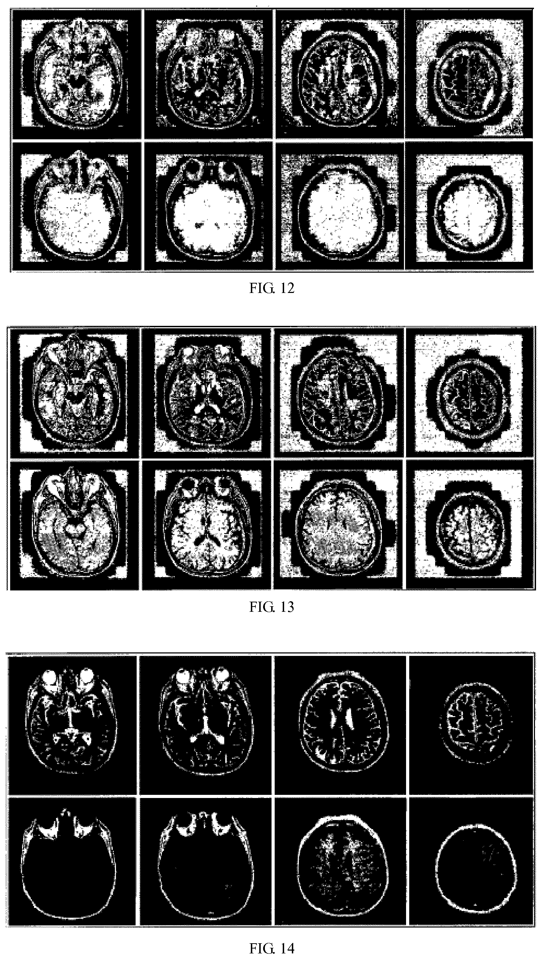

FIG. 12 showed that the hematoma of the patient is obviously absorbed two weeks upon the treatment, the midline shift was improved, and the hematoma amount was 80 ml.

FIG. 13 showed that the hematoma of the patient was mostly and significantly absorbed four weeks upon the treatment, the midline shift was disappeared, and the hematoma amount was 18 ml.

FIG. 14 showed that the hematoma of the patient absolutely disappeared in the patient continued to take atorvastatin for three months after stopping the drugs following four-week treatment.

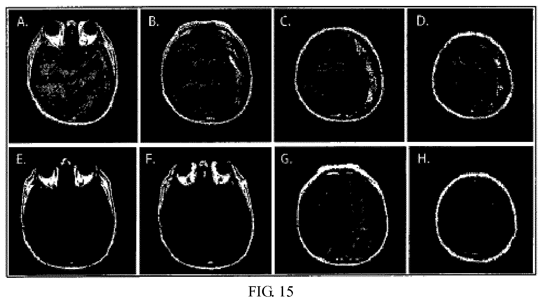

FIG. 15 was a comparison of head CT diagrams of a patient before and after treatment, wherein A-D showed that the amount of the chronic subdural hematoma before treatment was large, and the midline shift was obvious; and E-H showed that the hematoma of the patient absolutely disappeared in the patient continued to take atorvastatin for three months after stopping the drugs following four-week treatment.

6. Effect mechanism of treating chronic subdural hematoma by using the statins in combination with a small dose of dexamethasone

Statin, which is a hydroxymethylglutaryl coenzyme A reductase inhibitor, is currently widely confirmed as a drug that can promote angiogenesis after nerve injury, in addition to lowering blood lipid. It has been reported that the statins can promote the mobilization of circulating blood endothelial progenitor cells, and maintain endothelial progenitor cells at a higher level for more than 14 days. At the same time, atorvastatin has been verified to significantly inhibit VEGF, and can significantly reduce inflammation-related factors and inflammatory response. It has been further reported that the statins can also promote sustained enhancement of the expression of Notch1/Jagged1 signals, both of which are critical signals for regulating maturation of angiogenesis by VEGF. Recent animal experiments have found that a small dose of atorvastatin can affect the low expressions of capsular vessel-related factors VEFG, TGF-β, MMP-9, stabilize the Ang-1/Ang-2 ratio, and can significantly reduce the expression levels of local TNF-α and IL-6 gene and protein in the hematoma capsule. The above results showed that the mechanism of statins promoting the subdural hematoma absorption is associated with regulating local capsular angiogenesis of hematoma, promoting the maturation of neogenetic capsular vessels, inhibiting local abnormal inflammatory reactions and reducing vascular exudation and the like.

Dexamethasone can inhibit the formation of a new hematoma capsule in a rat model of subdural hematoma. Combined with the strong inflammation inhibition effect of glucocorticoid *per se,* the effects of the statins on inhibiting local abnormal inflammatory response and reducing vascular exudation and the like may be significantly enhanced. Based on the existing experimental theory, the mechanism of glucocorticoid in the treatment of chronic subdural hematoma is that glucocorticoid reduces the inflammatory reaction, thereby hindering the inflammatory cytokine-mediated abnormal angiogenesis, reducing plasma plasminogen and VEGF, and reducing vascular permeability. In addition, because of the strong local inflammation inhibitory effect, the glucocorticoid exerts a synergistic effect with statins and thereby achieves the effect of promoting the rapid absorption of hematoma at short notice by a small dose of dexamethasone.

## Claims

1. A combination of a statin and a glucocorticoid for use in a method of treating chronic subdural hematoma.

2. The combination of statin and glucocorticoid for use according to claim 1, wherein the statin is one or a combination of atorvastatin, simvastatin and rosuvastatin.

3. The combination of statin and glucocorticoid for use according to claim 1, wherein the glucocorticoid is one or a combination of dexamethasone, prednisone, methylprednisolone and hydrocortisone.

4. The combination of statin and glucocorticoid for use according to claim 1, wherein the glucocorticoid is dexamethasone or prednisone.

5. The combination of statin and glucocorticoid for use according to claim 1, wherein a mass ratio of the statin to the glucocorticoid in an equivalent dose is 40:3.

6. The combination of statin and glucocorticoid for use according to claim 5, wherein the statin is atorvastatin, and the glucocorticoid is dexamethasone, wherein a mass ratio of atorvastatin to dexamethasone is 40:3.

7. The combination of statin and glucocorticoid for use according to claim 6, wherein in an unit administration regimen, a dose of atorvastatin is 10 mg, and a dose of dexamethasone is 0.75 mg; or the dose of atorvastatin is 20 mg, and the dose of dexamethasone is 1.5 mg; or the dose of atorvastatin is 30 mg, and the dose of dexamethasone is 2.25 mg.

8. The combination of statin and glucocorticoid for use according to any one of claims 5 to 7, wherein administration doses of the statin and the glucocorticoid are respectively as follows:
30 mg/day, 2.25 mg/day; or
20 mg/day, 1.5 mg/day; or
10 mg/day, 0.75 mg/day;
wherein the doses can be administered as one administration per day or two to three administrations per day.

9. The combination of statin and glucocorticoid for use according to any one of claims 5 to 7, wherein administration doses of the statin and the glucocorticoid are respectively as follows:
administration in the first week: 30 mg/day, 2.25 mg/day;
administration in the second to the third week: 20 mg/day, 1.5 mg/day; or
administration in the fourth week: 10 mg/day, 0.75 mg/day.

10. The combination of statin and glucocorticoid for use according to claim 9, wherein an administration regimen of the statin and the glucocorticoid is as follows:
in the first week: atorvastatin 10 mg + dexamethasone 0.75 mg, 3 times per day;
in the second to the third week: atorvastatin 10 mg + dexamethasone 0.75 mg, 2 times per day; and
in the fourth week: atorvastatin 10 mg + dexamethasone 0.75 mg, 1 time per day.

## Patentansprüche

1. Kombination aus einem Statin und einem Glucocorticoid zur Verwendung in einem Verfahren zum Behandeln chronischer subduraler Blutergüsse.

2. Kombination aus Statin und Glucocorticoid zur Verwendung nach Anspruch 1, wobei das Statin eines oder eine Kombination aus Atorvastatin, Simvastatin und Rosuvastatin ist.

3. Kombination aus Statin und Glucocorticoid zur Verwendung nach Anspruch 1, wobei das Glucocorticoid eines oder eine Kombination aus Dexamethason, Prednison, Methylprednisolon und Hydrocortison ist.

4. Kombination aus Statin und Glucocorticoid zur Verwendung nach Anspruch 1, wobei das Glucocorticoid Dexamethason oder Prednison ist.

5. Kombination aus Statin und Glucocorticoid zur Verwendung nach Anspruch 1, wobei ein Massenverhältnis des Statins zu dem Glucocorticoid in einer Äquivalentdosis 40:3 beträgt.

6. Kombination aus Statin und Glucocorticoid zur Verwendung nach Anspruch 5, wobei das Statin Atorvastatin ist und das Glucocorticoid Dexamethason ist, wobei ein Massenverhältnis von Atorvastatin zu Dexamethason 40:3 beträgt.

7. Kombination aus Statin und Glucocorticoid zur Verwendung nach Anspruch 6, wobei in einem Einheitsverabreichungsschema eine Dosis von Atorvastatin 10 mg beträgt und eine Dosis von Dexamethason 0,75 mg beträgt; oder die Dosis von Atorvastatin 20 mg beträgt und die Dosis von Dexamethason 1,5 mg beträgt; oder die Dosis von Atorvastatin 30 mg beträgt und die Dosis von Dexamethason 2,25 mg beträgt.

8. Kombination aus Statin und Glucocorticoid zur Verwendung nach einem der Ansprüche 5 bis 7, wobei Verabreichungsdosen des Statins bzw. des Glucocorticoids wie folgt sind:
30 mg/Tag, 2,25 mg/Tag; oder
20 mg/Tag, 1,5 mg/Tag; oder
10 mg/Tag, 0,75 mg/Tag;
wobei die Dosen als eine Verabreichung pro Tag oder zwei bis drei Verabreichungen pro Tag verabreicht werden können.

9. Kombination aus Statin und Glucocorticoid zur Verwendung nach einem der Ansprüche 5 bis 7, wobei Verabreichungsdosen des Statins bzw. des Glucocorticoids wie folgt sind:
Verabreichung in der ersten Woche: 30 mg/Tag, 2,25 mg/Tag;
Verabreichung in der zweiten bis dritten Woche: 20 mg/Tag, 1,5 mg/Tag; oder
Verabreichung in der vierten Woche: 10 mg/Tag, 0,75 mg/Tag.

10. Kombination aus Statin und Glucocorticoid zur Verwendung nach Anspruch 9, wobei ein Verabreichungsschema des Statins und des Glucocorticoids wie folgt ist:
in der ersten Woche: Atorvastatin 10 mg + Dexamethason 0,75 mg, 3-mal pro Tag;
in der zweiten bis dritten Woche: Atorvastatin 10 mg + Dexamethason 0,75 mg, 2-mal pro Tag; und
in der vierten Woche: Atorvastatin 10 mg + Dexamethason 0,75 mg, 1-mal pro Tag.

## Revendications

1. Combinaison d'une statine et d'un glucocorticoïde pour une utilisation dans une méthode de traitement d'un hématome sous-dural chronique.

2. Combinaison de statine et de glucocorticoïde pour une utilisation selon la revendication 1, dans laquelle la statine est l'atorvastatine, la simvastatine ou la rosuvastatine, ou une combinaison de celles-ci.

3. Combinaison de statine et de glucocorticoïde pour une utilisation selon la revendication 1, dans laquelle le glucocorticoïde est la dexaméthasone, la prednisone, la méthylprednisolone ou l'hydrocortisone, ou une combinaison de celles-ci.

4. Combinaison de statine et de glucocorticoïde pour une utilisation selon la revendication 1, dans laquelle le glucocorticoïde est la dexaméthasone ou la prednisone.

5. Combinaison de statine et de glucocorticoïde pour une utilisation selon la revendication 1, dans laquelle un rapport massique de la statine au glucocorticoïde dans une dose équivalente est de 40:3.

6. Combinaison de statine et de glucocorticoïde pour une utilisation selon la revendication 5, dans laquelle la statine est l'atorvastatine, et le glucocorticoïde est la dexaméthasone, dans laquelle un rapport massique de l'atorvastatine à la dexaméthasone est de 40:3.

7. Combinaison de statine et de glucocorticoïde pour une utilisation selon la revendication 6, dans laquelle, dans un schéma d'administration d'une unité, une dose d'atorvastatine est de 10 mg, et une dose de dexaméthasone est de 0,75 mg ; ou la dose d'atorvastatine est de 20 mg, et la dose de dexaméthasone est de 1,5 mg ; ou la dose d'atorvastatine est de 30 mg, et la dose de dexaméthasone est de 2,25 mg.

8. Combinaison de statine et de glucocorticoïde pour une utilisation selon l'une quelconque des revendications 5 à 7, dans laquelle les doses d'administration de la statine et du glucocorticoïde sont respectivement les suivantes :
30 mg/jour, 2,25 mg/jour ; ou
20 mg/jour, 1,5 mg/jour ; ou
10 mg/jour, 0,75 mg/jour ;
dans laquelle les doses peuvent être administrées en une administration par jour ou deux ou trois administrations par jour.

9. Combinaison de statine et de glucocorticoïde pour une utilisation selon l'une quelconque des revendications 5 à 7, dans laquelle les doses de statine et de glucocorticoïde sont respectivement les suivantes :
administration pendant la première semaine : 30 mg/jour, 2,25 mg/jour ;
administration pendant la deuxième semaine jusqu'à la troisième semaine : 20 mg/jour, 1,5 mg/jour ; ou
administration pendant la quatrième semaine : 10 mg/jour, 0,75 mg/jour.

10. Combinaison de statine et de glucocorticoïde pour une utilisation selon la revendication 9, dans laquelle un schéma d'administration de la statine et du glucocorticoïde est le suivant :
pendant la première semaine : atorvastatine 10 mg + dexaméthasone 0,75 mg, 3 fois par jour;
pendant la deuxième jusqu'à la troisième semaine : atorvastatine 10 mg + dexaméthasone 0,75 mg, 2 fois par jour ; et
pendant la quatrième semaine : atorvastatine 10 mg + dexaméthasone 0,75 mg, 1 fois par jour.
